# EUROPÄISCHE PATENTSCHRIFT

(11) **EP 0 697 878 B1**
(45) Veröffentlichungstag und Bekanntmachung des Hinweises auf die Patenterteilung: **16.09.1998**
(21) Anmeldenummer: 94916915.5
(22) Anmeldetag: 02.05.1994
(51) Int. Cl.: A61K 35/78

(54) **NEUE VERWENDUNG VON EQUISETUM**
NEW USE OF EQUISETUM
NOUVELLE UTILISATION DE L'EQUISETUM

(30) Priorität: 02.05.1993 DE 4314131; 06.06.1993 DE 4318655
(43) Veröffentlichungstag der Anmeldung: 28.02.1996
(73) Patentinhaber: KÖNIGER, Helmut, D-80636 München (DE)
(72) Erfinder: KÖNIGER, Helmut, D-80636 München (DE)
(74) Vertreter: Turi, Michael, Dipl.-Phys.
(86) Internationale Anmeldenummer: EP9401383
(87) Internationale Veröffentlichungsnummer: WO9425041

(56) Entgegenhaltungen:
- WO-A-94/11009
- DD-A- 62 649
- DE-A- 4 244 754
- FR-A- 2 610 523

## Beschreibung

Die Erfindung betrifft die Verwendung von Bestandteilen von Pflanzen der Gattung Equisetum (Schachtelhalm), insbesondere der Art Equisetum arvense, zur topischen Bekämpfung von Psoriasis bzw. ein entsprechendes Behandlungsverfahren.

Für viele Erkrankungen sind nach wie vor keine zufriedenstellenden Arzneimittel bekannt, und in vielen Fällen, in denen wirksame synthetische Arzneistoffe erhältlich sind, weisen diese starke unerwünschte Nebenwirkungen auf. Deshalb besteht ein starkes Interesse daran, neue pflanzliche Heilmittel zu finden oder bekannte pflanzliche Heilmittel auf ihr gesamtes Wirkpotential zu untersuchen.

Equisetum, insbesondere die Arten Equisetum arvense (im Volksmund auch Zinnkraut genannt, da diese Art, die als Unkraut weit verbreitet ist, früher zum Reinigen von Zinngeräten verwendet wurde) und Equisetum hiemale, wurden vermutlich bereits im Altertum als Heilpflanzen verwendet. In neuerer Zeit wurde Equisetum vor allem durch Kneipp wieder in Erinnerung gebracht.

DD-62 649 verwendet einen Extrakt von herba Equiseti in Verbindung mit Haut- und Haarpflegemitteln, die Organpräparate enthalten, zur Geruchskompensierung von stark arteigen riechenden Organpräparaten. Die Verwendung von Equisetum-Bestandteilen in Form der pulverisierten Pflanze als schweißhemmendes Mittel ist in DE-A-32 07 005 beschrieben. Als hautstraffendes Mittel wird Equisetum arvense in Form eines wäßrigen oder glykolischen Extrakts in Seifen-Fette-Öle-Wachse, Zeitschrift für die Fett-, Öl-, Tensid-, Kosmetik- und Pharmaindustrie, 115(10), 1989, S. 331-338, Tabelle 6, Seite 337, erwähnt. Als Dekokt (10 g/1000 ml) haben Equiseti herba (equisetum arvense) eine Standardzulassung des Bundesgesundheitsamts zur Verwendung für Umschläge zur unterstützenden Behandlung schlecht heilender Wunden erlangt (Deutsche Apotheker Zeitung, 132 (Nr. 37), 1992, Seite 1876).

Die hauptsächlichen Inhaltsstoffe von Equisetum arvense (Kraut) sind Kieselsäure (zum Teil in löslicher Form), Equisetonin (Saponin), ein Bitterstoff, geringe Mengen 3-Methoxypyridin, Nicotin, Palustrin, Isoquercitrin, Galuteolin, Dimethylsulfon, Harz, Fett, Aconitsäure und andere Säuren, Vitamin C, Fermente und Polyensäuren.

Überraschenderweise hat sich nun herausgestellt, daß die Verwendung von Bestandteilen von Equisetum (Schachtelhalm), insbesondere Equisetum arvense (Ackerschachtelhalm), bevorzugt vorliegend als Pflanzensaft oder -extrakt mit einer Konzentration von 5-45 Vol.-% in einem zur topischen Behandlung annehmbaren Träger, auch bei der topischen Bekämpfung von Psoriasis wirksam ist.

Die Bekämpfung von Psoriasis ist dabei von ganz besonderem Interesse. Psoriasis war schon in der Antike bekannt, wurde aber erst Anfang des 19. Jahrhunderts von Robert Willan in England näher beschrieben. Mit einer Morbidität von 1%-2% in Deutschland, England und den Vereinigten Staaten von Amerika ist Psoriasis (vulgaris) (Schuppenflechte) eine der häufigsten und wichtigsten Hauterkrankungen. Eine genetische Prädisposition für die Krankheit ist belegt, eine spezifische Ursache dafür ist aber nach wie vor unbekannt.

Die Erkrankung äußert sich im Auftreten von scharf begrenzten, gesättigt weinroten Erythemen mit geschichteter, graumattsilberner Schuppung, und sie kann chronisch oder in Schüben verlaufen.

Die Behandlungsmöglichkeiten im akuten Stadium für diese sehr belastende Krankheit sind immer noch nicht als zufriedenstellend anzusehen. Eine systemische Behandlung ist nur bei ausgedehnten Krankheitsherden indiziert. Die topische Behandlung mit Anthralin, Teeren oder fluorierten Corticosteroiden bringt erhebliche Nachteile mit sich. Am wenigsten belastend ist eine Phototherapie, die aber gerade bei kleineren Krankheitsherden sehr zeitaufwendig sein kann, da in diesem Fall lokal eine photosensibilisierende Substanz angewendet wird, die eine Stunde einwirken muß, bevor bestrahlt werden kann. Bezüglich eines Überblicks über Psoriasis vulgaris und deren Therapie siehe O. Braun-Falco et. al., Dermatology, Springer Verlag, Berlin, 1991, Seiten 417 - 437.

Ein Medikament, das die Nachteile der bislang verwendeten topischen Medikamente nicht aufweist und ohne den Zeitaufwand, der für die Phototherapie erforderlich ist, zu einer Abheilung der Herde führt, wie es durch die vorliegende Erfindung bereitgestellt wird, ist also sehr wünschenswert, insbesondere wenn nur kleinere Krankheitsherde vorhanden sind.

Der biologische Wirkmechanismus, der den oben genannten Wirkungen von Equisetum zugrunde liegt, ist derzeit noch ungeklärt.

Definition: Unter dem Wort "Bekämpfung" wird in dieser Patentanmeldung die Vorbeugung gegen eine Erkrankung, die Linderung einer Erkrankung und die Heilung einer Erkrankung verstanden.

Die Arten Equisetum arvense und Equisetum hiemale werden bevorzugt als Ausgangspflanzen bei der erfindungsgemäßen Verwendung eingesetzt.

Eine ganze Reihe von Aufbereitungsformen der Pflanzen der Gattung Equisetum sind für eine zur Anwendung geeignete Zubereitung möglich, z.B. die ganze Pflanze in pulverisierter Form, ein aus den Wurzeln, der ganzen grünen Pflanze oder dem Kraut der Pflanze ausgepreßter Saft, ein wäßriger Aufguß von Equisetum oder ein wäßrig-alkoholischer Extrakt aus dem Kraut. Eine hervorragende Übersicht über mögliche Aufbereitungsformen, die in diesem Zusammenhang verwendet werden können, findet sich in Paul Heinz List und Peter C. Schmidt, "Technologie pflanzlicher Arzneizubereitung", Wissenschaftliche Verlagsgesellschaft mbH, Stuttgart, 1984. Als Ausgangsmaterial für die vorliegende Erfindung wird derzeit bevorzugt ein ethanolisch-wäßriger (30:70 Vol/Vol) Fluidextrakt (1:1 Vol/Vol) von Schachtelhalmkraut verwendet.

Dieses Ausgangsmaterial kann unter Umständen als solches für die topische Behandlung eingesetzt werden, wird aber bevorzugt in eine für die äußerliche Anwendung geeignete, weitere Stoffe enthaltende galenische Form, wie z.B. Puder, Salbe, Creme, Gel, Lotion, Emulsion, Lösung, beispielsweise als Spray auftragbar, und Spray mit Treibmittel (Aerosole), gebracht. Eine Übersicht über mögliche Galeniken ist z.B. in Karl Thoma, "Dermatika", 2. Auflage, München, 1983 (zu beziehen über Werbe- und Vertriebsgesellschaft Deutscher Apotheker m.b.H., Frankfurt) sowie in Remington's Pharmaceutical Sciences, 18. Auflage, Mack Publishing Company, Easton, Pennsylvania, 1990, insbesondere auch Seiten 1694 - 1712 (Aerosole) zu finden. Die Konzentration an Equisetum-Inhaltsstoffen in diesen galenischen Formen bewegt sich gewöhnlich im Bereich von ungefähr 0,1 bis ungefähr 45 Gewichts- bzw. Volumenprozent, bevorzugt von ungefähr 5 bis 45 Volumenprozent und insbesondere ungefähr 5 bis 20 Volumenprozent.

Pharmazeutische Zusammensetzungen, die Equisetumsaft oder -extrakt zu 5-45 Volumenprozent enthalten, sind neu, solange sie kein Organpräparat (z.B. Placentaextrakt, Keratinhydrolysat, Bakterien- und Hefeextraktfiltrat) enthalten. Pharmazeutische Zusammensetzungen mit dieser Konzentration an Equisetum haben sich als sehr wirksam erwiesen, ohne daß durch die relativ hohe Konzentration eine andere Nebenwirkung als ein gelegentliches Trockenheitsgefühl an den behandelten Hautstellen auftrat.

Als besonders geeignete galenische Form für die erfindungsgemäße Verwendung hat sich eine Lösung oder Emulgation eines wie oben zusammengesetzten ethanolischwäßrigen Fluidextrakts von Equisetum in einer Mischung von Ethanol oder Isopropanol und Wasser, insbesondere 25-35% (Vol/Vol) Isopropanol/Wasser, die bequem mit Hilfe eines Zerstäubers aufgetragen werden kann, als sehr brauchbar erwiesen. Die Konzentration des Extrakts, ausgedrückt als unverdünnter Extrakt ohne Extraktionsmittel-Anteil, liegt in dieser galenischen Form für die meisten Anwendungsgebiete vorteilhafterweise zwischen 5 und 45% (Vol/Vol), wobei in einigen Fällen auch geringere oder höhere Konzentrationen vorteilhaft sein können.

Die für die erfindungsgemäße Verwendung eingesetzten Zusammensetzungen können neben Trägerstoffen, Emulgatoren und anderen pharmazeutischen Hilfsstoffen, z.B. rückfettenden Mitteln, auch noch weitere Beistoffe enthalten, wie beispielsweise natürliche Duftstoffe oder weitere medizinische, auch phytotherapeutische, Mittel. Erfindungsgemäß bevorzugt verwendete Zusammensetzungen können vorteilhafterweise zusätzlich zu Bestandteilen von Pflanzen der Gattung Equisetum Bestandteile von Pflanzen der Gattung Lavandula, insbesondere Lavendelöl und/oder einen Lavendelextrakt, als weiteres phytotherapeutisches Mittel enthalten, das aufgrund von bisher ungeklärten Mechanismen die Wirkung des Equisetum verstärken kann, und/oder einen Emulgator, vorzugsweise einen nichtionischen Emulgator wie beispielweise Tween^{(R)} (Polyethoxysorbitanstearat), z.B. Tween^{(R)} 60. Dabei beträgt das Verhältnis von Equisetum-Bestandteilen zu Lavendel-Bestandteilen im allgemeinen 90 : 10 bis 10 : 90 (Vol./Vol.). Die Konzentration an Emulgator wird so gehalten, daß sich stabile Emulsionen bilden.

Da Equisetum unter Umständen eine austrocknende Wirkung aufweisen kann, kann es empfehlenswert sein, nach dem Auftragen eines Equisetum-haltigen Mittels die Haut mit einer wasserfreien Fettsalbe zu behandeln, falls das Mittel selbst nicht schon eine rückfettende Substanz enthält.

Die Häufigkeit und die Dauer der Auftragung eines erfindungsgemäß verwendeten Equisetum-haltigen Mittels richtet sich nach dessen Wirkstoff-Konzentration und der beabsichtigten Wirkung, also danach, ob eine vorbeugende Wirkung oder die Bekämpfung eines akuten Krankheitszustandes, wobei die Schwere der Erkrankung eine Rolle spielt, erreicht werden soll.

Im folgenden werden beispielhaft einige typische Applikationshäufigkeiten und -dauern aufgeführt, die aber keinesfalls als beschränkend anzusehen sind; auch andere als die erwähnten Applikationshäufigkeiten und -dauern liegen im Rahmen der Erfindung, ebenso die Verwendung von anderen als den erwähnten Konzentrationen an Equisetum-Bestandteilen.

Zur Bekämpfung von Psoriasis wird beispielsweise drei- bis viermal täglich ein Equisetum-haltiges Medikament auf die befallene(n) Stelle(n) aufgetragen, bis jegliche Rötung verschwunden ist, wobei diese Zeitdauer von Fall zu Fall sehr stark schwanken kann. Es hat sich als vorteilhaft erwiesen, anschließend eine dünne Schicht einer wasserfreien Fettsalbe aufzubringen.

Eine Entschuppung vor und während der Behandlung von Psoriasis (z.B. mit Salicylsäure) mit Equisetum-haltigen Arzneimitteln ist häufig nicht unbedingt erforderlich, da dieses Arzneimittel selbst entschuppend wirken kann.

Das Auftragen des Equisetum-haltigen Mittels geschieht, wenn es sich um die oben beschriebene Form eines Equisetum-Extraktes in wäßrigem Isopropanol als Träger handelt, geeigneterweise mittels einer Sprühvorrichtung oder mittels eines getränkten Wattebausches, bis sich ein dünner Film auf der Haut gebildet hat; gegebenenfalls wird die aufgetragene Flüssigkeit mit der Hand gleichmäßig verteilt. Anschließend läßt man den Flüssigkeitsfilm eintrocknen und trägt gegebenenfalls eine wasserfreie Fettsalbe auf.

Die Erfindung wird nun anhand von insbesondere hinsichtlich Konzentrationen und Applikationshäufigkeiten und -dauern nicht-beschränkenden Beispielen näher erläutert.

### Verwendete Ausgangsmaterialen

Bestandteile des Krauts von Equisetum arvense wurden in Form eines handelsüblichen Extrakts in den in den Beispielen angegebenen Konzentrationen eingesetzt (Schachtelhalmkraut-Fluidextrakt, Fluidextrakt 1:1 (Vol/Vol), Auszugsmittel 30%-iges( Vol/Vol) wäßriges Ethanol, erhältlich von Dr. Hetterich KG, Fürth; im folgenden als "handelsüblicher Equisetum-Extrakt" bezeichnet).

Das eingesetzte Lavendelöl war ein handelsübliches Produkt.

Als Trägermittel wurde 25, 30 oder 35%iges (Vol./Vol.) wäßriges Isopropanol verwendet.

Falls nicht anders angegeben, wurden den Zusammensetzungen zur Stabilisierung der Emulsion 1 bis 3 Spatelspitzen Tween ^{(R)} 60 zugesetzt.

Zum Auftragen der Zusammensetzung wurde ein Behälter mit einer Sprühvorrichtung verwendet.

### Beipiele 1 - 3

### Bekämpfung von Psoriasis

### Verwendete Zusammensetzungen:

I: 20%ige (Vol./Vol.) Emulsion des handelsüblichen Equisetum-Extrakts in 30%igem wäßrigem Isopropanol.
II: 2,6%ige (Vol./Vol.) Emulsion des handelsüblichen Equisetum-Extrakts in 30%igem wäßrigem Isopropanol.

In keinem der Beispiele 1A-1C wurde eine gesonderte Entschuppungsbehandlung durchgeführt.

### Beispiel 1:

Ein männlicher Proband mit Psoriasis auf beiden Handrücken (pergamentartige Haut), beiden Ellenbogen (ca. 25 cm langer Herd) und am Hinterkopf, deren Schuppen zu Behandlungbeginn nicht entfernt waren, sprühte in täglichem Wechsel jeweils morgens und abends einmal die obigen Zusammensetzungen I und II auf die Krankheitsherde, bis ein zusammenhängender Film entstanden war. Nach Einziehen des Mittels wurden die befallenen Stellen zur Durchblutungserhöhung solange geklopft, bis die Rötung weiß wurde. Anschließend wurde eine wasserfreie Salbenbasis dünn aufgetragen.

Nach zweimonatiger Behandlung war der Herd am Hinterkopf völlig verschwunden, ebenso auf den Handrücken, deren Haut wieder eine normale Beschaffenheit aufwies (lediglich am Knöchel der Ringfingers der rechten Hand war noch eine minimale Rötung zu beobachten, vermutlich bedingt durch vieles Schreiben). An den Ellenbogen trat keine Schuppung mehr auf. Es war lediglich noch eine ca. 5 bis 6 cm lange hellrosafarbene Rötung zu beobachten.

### Beispiel 2:

Ein männlicher Proband mit Psoriasis im Haarbereich, die 1/2 Jahr auf herkömmliche Weise erfolglos behandelt worden war, behandelte zweimal täglich die befallenen Stellen mit der obigen Zusammensetzung I (eine wasserfreie Salbe wurde nicht verwendet). Nach 2 Wochen waren keine Schuppen und keinerlei Rötung mehr vorhanden.

### Beispiel 3:

Eine weibliche Testperson mit relativ schwerer Psoriasis an den Händen, Ellenbogen, Knien und Füßen wies zu Beginn der Behandlung die folgenden Symptome auf:
Ellenbogen (ca. 15 cm langer Herd), Handrücken, Knie: rote Herde mit weißlichen Schuppen;
Handinnenflächen und Fußsohlen: ca. 2 mm dicke hornhautähnliche Schicht;
Zehenzwischenräume: hornhautähnliche Schicht;
Fingeroberseite: rote, pergamentartige Haut;
Handinnenflächen ohne Tastgefühl; Hände morgens kaum beweglich, da die Haut wie "Blech" ist.

Nach 7monatiger Behandlung, die wie im Beispiel 1A durchgeführt wurde, ergab sich folgendes Bild:
Knie, Zehenzwischenräume: völlig ausgeheilt; Ellenbogen und
Handrücken: jeweils nur noch an zwei sehr kleinen Flächen etwas Rötung.
Fußsohlen: Haut wieder normal, nur noch ganz leichte Rötung;
Handflächen: bedeutend weniger hornhautartige Haut; Tastgefühl und Beweglichkeit am Morgen wieder vorhanden;
Fingeroberseite: bedeutender Rückgang der Rötung an den beiden mittleren Fingern und am Daumen.

## Patentansprüche

1. Verwendung von Bestandteilen von Pflanzen der Gattung Equisetum zur Herstellung eines Medikaments zur topischen Bekämpfung von Psoriasis.

2. Verwendung nach Anspruch 1, dadurch gekennzeichnet, daß die Pflanzen den Arten Equisetum arvense und/oder Equisetum hiemale angehören.

3. Verwendung nach einem der Ansprüche 1 bis 2, dadurch gekennzeichnet, daß der Saft oder ein Extrakt der Pflanzen verwendet wird.

4. Verwendung nach Anspruch 3, dadurch gekennzeichnet, daß ein wäßrig-alkoholischer Extrakt der Pflanzen, der in einem zur topischen Behandlung geeigneten Träger, gegebenenfalls zusammen mit weiteren Beistoffen, vorliegt, verwendet wird.

5. Verwendung nach den Ansprüchen 1 bis 4, dadurch gekennzeichnet, daß die Konzentration an Equisetum-Bestandteilen 5 bis 45 Gew.- oder Vol.-%, bezogen auf das Gesamtgewicht oder -volumen der verwendeten Equisetumhaltigen Zusammensetzung, beträgt.

6. Verwendung nach den Ansprüchen 2 bis 4, dadurch gekennzeichnet, daß die Konzentration an Equisetum-Bestandteilen 0,1 bis 2 Gew.- oder Vol.-%, bezogen auf das Gesamtgewicht oder -volumen der verwendeten Equisetumhaltigen Zusammensetzung, beträgt.

7. Verwendung nach einem der Ansprüche 1 bis 6, dadurch gekennzeichnet, daß Equisetum-Bestandteile und Lavendelöl und/oder ein Lavendelextrakt zusammen verwendet werden.

8. Verwendung nach einem der Ansprüche 1 bis 7, dadurch gekennzeichnet, daß neben den Equisetum-Bestandteilen auch ein Emulgator verwendet wird.

## Claims

1. Use of elements of plants of the genus Equisetum for the manufacture of a medication for the topical treatment of psoriasis.

2. Use according to Claim 1, characterised in that the plants belong to the types Equisetum arvense and/or Equisetum hiemale.

3. Use according to one of Claims 1 to 2, characterised in that the sap or an extract of the plants is used.

4. Use according to Claim 3, characterised in that an aqueous alcoholic extract of the plants, in a carrier which is suitable for topical treatment, if necessary together with further additional substances, is used.

5. Use according to Claims 1 to 4, characterised in that the concentration of Equisetum elements is 5 to 45 percent by weight or by volume in relation to the overall weight or volume of the Equisetum-containing composition used.

6. Use according to Claims 2 to 4, characterised in that the concentration of Equisetum elements is 0.1 to 2 percent by weight or by volume in relation to the overall weight or volume of the Equisetum-containing composition used.

7. Use according to one of Claims 1 to 6, characterised in that the Equisetum elements and lavender oil and/or lavender extract are used in combination.

8. Use according to one of Claims 1 to 7, characterised in that an emulsifying agent is used along with the Equisetum elements.

## Revendications

1. Utilisation de composants de plantes du genre Equisetum pour la préparation d'un médicament destiné au traitement topique du psoriasis.

2. Utilisation selon la revendication 1, caractérisée en ce que les plantes appartiennent aux espèces Equisetum arvense et/ou Equisetum hiemale.

3. Utilisation selon la revendication 1 ou 2, caractérisée en qu'on utilise la sève ou un extrait des plantes.

4. Utilisation selon la revendication 3, caractérisée en ce qu'on utilise un extrait d'alcool aqueux des plantes, qui se présente dans un véhicule approprié pour un traitement topique, éventuellement conjointement à d'autres additifs.

5. Utilisation selon les revendications 1 à 4, caractérisée en ce que la concentration en composants d'Equisetum est comprise entre 5 et 45 % en masse ou en volume, rapporté à la masse totale ou au volume total de la composition à base d'Equisetum utilisée.

6. Utilisation selon les revendications 2 à 4, caractérisée en ce que la concentration en composants d'Equisetum est comprise entre 0,1 et 2 % en masse ou en volume, rapporté à la masse totale ou au volume total de la composition à base d'Equisetum utilisée.

7. Utilisation selon l'une des revendications 1 à 6, caractérisée en ce qu'on utilise conjointement des composants d'Equisetum et de l'huile de lavande et/ou un extrait de lavande.

8. Utilisation selon l'une des revendications 1 à 7, caractérisée en ce qu'on utilise également, en sus des composants d'Equisetum, un émulsifiant.
